# FASCICULE DE BREVET EUROPEEN

(11) **EP 3 525 066 B1**
(45) Date de publication et mention de la délivrance du brevet: **08.05.2024**
(21) Numéro de dépôt: 19156219.8
(22) Date de dépôt: 08.02.2019
(51) Int. Cl.: G06F 3/01, G02B 27/01, A61B 3/00, A61B 3/024, A61B 3/113, B64D 43/00

(54) **VISION PÉRIPHÉRIQUE DANS UNE INTERFACE HOMME-MACHINE**
PERIPHERISCHES SEHEN IN EINER MENSCH-MASCHINE-SCHNITTSTELLE
PERIPHERAL VISION IN A HUMAN MACHINE INTERFACE

(30) Priorité: 12.02.2018 FR 1800125
(43) Date de publication de la demande: 14.08.2019
(73) Titulaire: Thales, 92190 Meudon (FR)
(72) Inventeur: LAFON, Stéphanie, 33700 Merignac (FR); BAILLY, Alexiane, 33700 Merignac (FR); DOTTE, Sébastien, 33700 Merignac (FR)
(74) Mandataire: Atout PI Laplace

(56) Documents cités:
- WO-A1-2017/031089
- FR-A1- 3 046 225
- US-A1- 2010 156 617
- US-A1- 2016 272 340
- US-A1- 2017 160 546

## Description

### Domaine de l'invention

L'invention concerne le domaine technique des procédés et des systèmes d'affichage en général, et plus spécifiquement celui des interfaces multimodales au sein d'un cockpit d'aéronef.

### Etat de la Technique

Dans la cabine de pilotage d'un aéronef contemporain, le pilote reçoit et manipule une grande quantité d'informations. Dans certaines circonstances (e.g. contexte de vol ou type de mission), le pilote peut être surchargé d'informations au point de ne pas répondre à des sollicitations communiquées par les systèmes avioniques. Dans certains cas, le pilote peut ne pas même percevoir les stimuli. En effet, un pilote surchargé ou distrait par d'autres tâches peut ne pas relever une information importante que le système lui présenterait. Dans l'histoire aéronautique, il est déjà arrivé qu'un pilote, sous l'effet du stress, ne perçoive pas une alerte audio. Les conséquences peuvent se révéler être dramatiques.

Par ailleurs, les interfaces homme-machine se complexifient de manière croissante. Aux fins notamment de la sécurité et/ou de la sûreté aéronautique, il existe donc un besoin toujours croissant de gestion améliorée des interfaces homme-machine (au sens large, c'est-à-dire de manière multimodale i.e. faisant intervenir différentes capacités sensorielles).

Les systèmes avioniques existants informent les pilotes de défaillance système par le biais d'alertes sonores ou de messages vocaux spécifiques. Des indicateurs lumineux additionnels sont parfois utilisés, en redondance. Lorsqu'il reçoit un message de type *datalink,* par exemple en provenance du contrôle de la navigation aérienne, un signal audio peut être émis dans le cockpit et des interfaces homme-machine spécialisées peuvent signaler l'arrivée des messages. Le pilote peut alors réagir, notamment par interaction tactile avec les équipements d'interface disposés dans le cockpit (e.g. envoi de messages au moyen d'un clavier physique. Ces solutions existantes présentent des défauts ou des insuffisances.

La littérature publiée sur les interfaces homme-machine est riche mais les spécificités aéronautiques demeurent partiellement vierges, au moins sur des aspects récents.

Le document de brevet US2016272340 intitulé « *Aircraft-vision systems and methods for maintaining situational awareness and spatial orientation* » divulgue des systèmes et des procédés d'affichage pour maintenir la conscience de la situation et l'orientation spatiale du pilote, en particulier dans des conditions difficiles. Une image avec des repères visuels est projetée dans une zone périphérique du champ visuel de sorte que ces repères stimulent la vision périphérique du pilote. Cette approche présente des limitations.

Le document de brevet US2010156617 intitulé "Apparatus, method and program for driving attention amount détermination" divulgue un système d'assistance à la conduite d'un véhicule affichant une interface homme-machine en fonction de données physiologiques mesurées sur le conducteur. Cette approche présente également des limitations.

Il existe un besoin pour des systèmes et des procédés de gestion de l'affichage en périphérie du champ de vision.

### Résumé de l'invention

La présente invention est définie dans l'énoncé des revendications indépendantes 1, 9 et 10. Certains modes de réalisation avantageux sont définis dans l'énoncé des revendications dépendantes.

L'invention concerne un procédé mis en oeuvre par ordinateur de gestion d'une interface graphique homme-machine, comprenant les étapes consistant à recevoir des informations relatives à la position des yeux et la direction du regard d'un utilisateur sur l'interface; recevoir des informations physiologiques de l'utilisateur ; déterminer un niveau de charge cognitive en fonction des informations physiologiques reçues ; ajuster l'affichage de l'interface en fonction de la direction du regard et/ou du niveau de charge cognitive déterminée. Des développements décrivent la gestion des zones d'affichage (zone fovéale et zones périphériques), la sélection d'un ou de plusieurs afficheurs, la gestion de la distance de l'affichage de messages à l'emplacement courant du regard, la gestion de la criticité des messages, diverses modalités graphiques pour attirer l'attention, la gestion du contexte de vol dans le cas avionique, la gestion de la densité visuelle, etc. Des aspects de système sont décrits (réalité virtuelle et/ou augmentée).

De manière générale, les exemples fournis facilitent les interactions homme-machine et en particulier déchargent le pilote de manipulations fastidieuses, parfois répétitives et souvent complexes, améliorant du même coup sa capacité de concentration pour le pilotage proprement dit. Ces manipulations et opérations se déroulent fréquemment dans des contextes d'urgence ou demandant une réaction rapide. L'effort cognitif à fournir pour la conduite ou le pilotage se trouve optimisé, ou plus exactement réalloué à des tâches cognitives plus utiles au regard de l'objectif de pilotage. En d'autres termes, les effets techniques liés à certains aspects de l'invention correspondent à une réduction de la charge cognitive de l'utilisateur de l'interface homme- machine.

Les améliorations et perfectionnements selon l'invention sont avantageux en ce qu'ils réduisent les risques de défaillances humaines dans le cadre de l'interaction homme-machine (e.g. risque de suppression ou de non-prise en compte de données critiques etc.). Les modes de réalisation de l'invention contribuent donc à améliorer la sécurité et la sûreté aéronautique (plus généralement du pilotage de véhicule).

Certains modes de réalisation permettent d'alerter ou d'informer le pilote, de manière visuelle quant à un évènement particulier *en fonction de la charge cognitive* qu'il subit (e.g. sans le perturber dans leur tâche courante). Par contraste, les solutions existantes sont figées ou statiques au sens où elles ne prennent pas en compte la charge cognitive du pilote (une alerte sonore peut ne pas être perçue lors d'une forte charge de travail, e.g. un message ou un témoin lumineux peut sortir du champ visuel du pilote car la vision périphérique peut se réduire).

Par ailleurs, certains modes de réalisation permettent d'alerter ou d'informer le pilote, de manière visuelle quant à un évènement particulier, *quel que soit l'endroit où il regarde.* Les modes de réalisation de l'invention optimisent donc la sollicitation et l'utilisation du champ de vision du pilote.

Dans certains modes de réalisation avantageux, l'interface homme-machine selon l'invention est partiellement ou totalement contrôlable avec le regard. Par exemple, le pilote peut interagir avec l'interface sans les mains et avec son seul regard. Il peut par exemple répondre (confirmer ou infirmer) à une question ou une demande de l'interface. Il peut également initier une action. Cette modalité d'interaction est plus sûre en ce qu'elle permet au pilote de conserver ses mains sur les commandes de l'aéronef. Par contraste, les approches connues de l'état de la technique nécessitent *a minima* des interventions tactiles alors qu'un pilote peut parfois ne pas pouvoir utiliser ses mains (lorsqu'il tient les commandes de son aéronef par exemple).

En élargissant les méthodes d'affichage aux environnements de réalité augmentée et/ou virtuelle, l'invention permet de nouvelles possibilités d'interaction. Par contraste, les interfaces homme-machine selon l'état de la technique en avionique sont généralement conçues en rapport avec les formats d'affichage (limités voir exigus). De nouvelles possibilités d'interaction augmentée ou virtuelle permettent de redéfinir les interfaces homme-machine elles-mêmes. Par exemple, le champ visuel de l'utilisateur peut être utilisé au mieux et de manière plus intensive. Un véritable dialogue interactif entre la machine et l'homme peut se dérouler, par exemple pour maintenir un niveau d'attention élevé, ou exploiter ce dernier au mieux. En l'espèce, les modes de réalisation de l'invention permettent d'augmenter la surface d'affichage utilisée ou utilisable en périphérie du champ de vision, en optimisant l'utilisation de cette zone visuelle.

Dans un mode de réalisation avantageux de l'invention, le meilleur endroit pour afficher de l'information est sélectionné en temps réel, notamment en fonction du regard du pilote.

Dans un mode de réalisation avantageux de l'invention, une icône mobile ou symbole est affiché dans le champ périphérique du pilote, à une distance dépendante de sa charge cognitive.

Dans un mode de réalisation avantageux de l'invention, l'interface est contrôlable du regard.

### Description des figures

Différents aspects et avantages de l'invention vont apparaitre en appui de la description d'un mode préféré d'implémentation de l'invention mais non limitatif, avec référence aux figures ci-dessous :
La figure 1 illustre un exemple d'interface homme-machine dans le contexte particulier de l'avionique, interface qui est manipulée par le procédé selon l'invention ;
La figure 2 illustre un système d'affichage spécifique;
La figure 3 montre des exemples d'étapes du procédé selon l'invention;
La figure 4 détaille un mode de réalisation particulier ;
La figure 5 illustre un exemple de gestion d'un message selon un mode de réalisation particulier de l'invention.

### Description détaillée

Selon les modes de réalisation de l'invention, un aéronef peut être un avion commercial, militaire ou de fret, un drone autonome ou télé piloté, un hélicoptère, ou tout autre moyen de transport pouvant utiliser une interface homme-machine.

Le terme « dispositif d'affichage » manipulé par l'invention désigne tout système d'affichage intercalé ou interposé entre le système visuel (i.e. les yeux) d'un utilisateur (e.g. chirurgien, pilote, informaticien, etc.) et son environnement extérieur (lequel procure donc un « fond visuel »).

Le « champ visuel » désigne littéralement la portion de l'espace vue par un oeil. Un oeil perçoit dans cette zone d'espace des lumières, des couleurs, des formes, des textures, etc. Par extension, les deux yeux perçoivent en stéréoscopie une portion d'espace. La vision peut être de différents types : monoculaire ou binoculaire. Ce champ visuel comporte plusieurs zones. L'acuité maximale correspond à la zone fovéale ; d'autres zones permettent la lecture, la reconnaissance des symboles, et la discrimination des couleurs. Le champ de vision d'un pilote évolue avec les mouvements oculaires : par exemple, le champ de vision se rétrécit avec la vitesse (signifiant en fait que les yeux deviennent moins mobiles).

Dans la suite du document, l'expression « emplacement du regard » désigne le centre de la vision fovéale (centre de la zone perçue par le cône de vision).

La vision « périphérique » désigne une caractéristique de la vision humaine. Concernant la vision fovéale, l'oeil s'arrête pendant 200 à 400 millisecondes sur un point de fixation afin d'obtenir des détails à haute résolution. La vision fovéale procure une analyse détaillée mais lente (3 à 4 « images » par seconde). Par contraste, la vision périphérique procure des impressions plus globales (voire déformées) du champ de vision mais très rapides (jusqu'à 100 « images » par seconde). La vision périphérique permet donc la perception ultra-rapide de mouvements. Cette capacité de détecter des mouvements augmente même vers la périphérie extrême. Il est estimé que la vision périphérique couvre plus de 99 % du champ de vision et est associées à la moitié du nerf optique et du cortex visuel.

L'acuité visuelle est maximale dans la zone fovéale (5° de FOV pour « Field of View », angle solide selon la direction du regard). La lecture s'opère généralement dans un intervalle excédant la zone fovéale (10° FOV). La reconnaissance des symboles s'effectue dans un intervalle de 20° FOV et la discrimination des couleurs est généralement effectuée selon un intervalle de 30° FOV.

La vision est dite maculaire jusqu'à 18° FOV, puis au-delà la vision est dite périphérique. Cette vision périphérique peut se décomposer en périphérie « proche », « moyenne » ou « lointaine » (respectivement « *near*, *mid*, *far peripheral* » en anglais), selon différents seuils (généralement 60° et 120° FOV). L'expression « vision périphérique » dans ce document désigne ces différentes zones.

La périphérie du champ de vision est *relative* par rapport à la position de la tête de l'utilisateur. La tête pouvant bouger, s'orienter (e.g. sur les côtés, vers le bas, vers le haut), il est implicite dans le reste du document que le suivi de la tête du pilote (« head-tracking » en anglais) peut être effectué (si les particularités géométriques de vision le nécessitent).

La zone fovéale et les zones de vision périphériques s'inscrivent dans le champ visuel (subjectif), lequel comprend plusieurs composantes indépendantes ou dépendantes : le fond visuel (environnement lointain e.g. montagnes, nuages, route, etc.), les afficheurs à positions fixes dans le véhicule (mais qui peuvent bouger par rapport à la position de la tête de l'utilisateur) et les afficheurs portés par l'utilisateur (opaques, transparents ou semi-transparents), lesquels sont généralement solidaires de la tête de l'utilisateur.

Dans les modes de réalisation dans lesquels l'interface homme-machine est au moins partiellement portée (e.g. casque de réalité virtuelle RV ou de réalité augmentée RA, transparent ou semi-transparent), la zone fovéale et les zones périphériques bougent avec la direction du regard. Dans ce type d'affichage RV/RA, le fond visuel (i.e. les différentes plans ou profondeurs d'affichage) peut être modifié ou ajusté (par comparaison avec un extérieur naturel et invariant à l'horizon lorsque la RV ou RA n'est pas utilisée). En d'autres termes, dans ce type d'environnements RV/RA, il existe une ou plusieurs « couches » ou profondeurs d'affichage intermédiaires, artificielles (configurables et ajustables), intercalées entre la réalité et le système visuel, qui peuvent être manipulées de manière à gérer cette zone fovéale et ces zones périphériques. Par exemple, le rendu graphique (« *rendering* » graphique i.e. précision et qualité photométrique d'images, nombre de polygones, temps de latence et rafraichissement graphique, etc.) peut être particulièrement optimisé pour la zone fovéale, tandis que les zones périphériques peuvent solliciter moins de puissance de calcul. Dans d'autres scénarios, les compromis peuvent être différents, voire inverses.

Dans certains modes de réalisation, l'environnement visuel extérieur peut être perçu par transparence (en plus d'un afficheur porté par l'utilisateur, des éléments du tableau de bord du cockpit peuvent être perçus). Autrement dit, des éléments virtuels autant que des éléments « réels » peuvent être perçus.

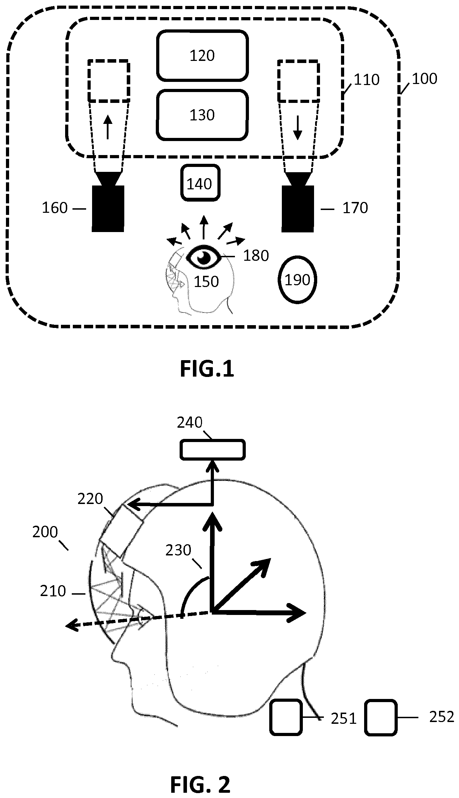

La figure 1 illustre un exemple d'interface homme-machine dans le contexte particulier de l'avionique, interface qui est manipulé par le procédé selon l'invention.

Le cockpit 100 d'un aéronef (ou d'une cabine de télé-pilotage, ou d'un véhicule au sens large) peut comprendre un tableau de bord 110 comprenant un ou plusieurs affichages. Une interface homme-machine selon l'invention peut comprendre plusieurs systèmes d'affichage par exemple disposés sur un tableau de bord ou dans le cockpit et/ou portés par le pilote. Un système d'affichage ou interface homme-machine selon l'invention peut comprendre un ou plusieurs affichages (« écrans » ou « afficheurs ») choisis parmi un affichage tête-haute 120, un affichage tête-basse 130, un écran portable ou transportable 140, un affichage en réalité virtuelle et/ou augmentée 150, un ou plusieurs projecteurs 160. L'interface homme-machine peut également comprendre une caméra ou dispositif d'acquisition d'images 170 ainsi que des interfaces ou périphériques d'entrée 180.

Un affichage tête-haute 120 peut être de type HUD (en anglais « Head Up Display », HUD). Un affichage « tête haute » ou « tête portée » permet au pilote d'un véhicule de surveiller son environnement en même temps que les informations fournies par ses instruments de bord. Ce type de dispositif superpose des informations particulières (e.g. pilotage, navigation, mission) dans le champ de vision du pilote. Différents dispositifs sont connus dans l'état de la technique, notamment l'utilisation de projecteurs, de miroirs semi-transparents, de surfaces de projection transparentes (e.g. réalité augmentée) ou opaques (e.g. réalité virtuelle), voir dont l'opacité est configurable. Dans certains modes de réalisation, un affichage tête-haute est de type binoculaire (i.e. sollicite les deux yeux). Dans d'autres cas, l'affichage est de type monoculaire (i.e. mobilisant un seul oeil). Dans de tels systèmes à tête portée monoculaire, des informations sont surimposées à l'environnement. Un système HUD peut donc être de type monoculaire (e.g. lunettes connectées Google Glass), mais également de type binoculaire (e.g. un système « *Head-Mounted Display* » ou HMD qui délivre deux images différentes, une pour chaque oeil). Un système d'affichage dans certain cas peut aussi être « bi-oculaire » (présentant les mêmes images aux deux yeux). Un pare-brise sur lequel sont projetées des informations de conduite procure des images différentes à l'oeil droit et à l'oeil gauche et entre donc dans la catégorie des systèmes d'affichage binoculaires.

Un affichage tête-basse 130 est un affichage disposé en dessous d'une zone de vision principale. Un affichage tête-basse utilisé par l'invention peut être par exemple un écran principal de vol PFD (et/ou un écran de navigation ND/VD et/ou un écran multifonction MFD). Plus généralement, les écrans peuvent être des écrans avioniques de type de gestion de vol ou « *Flight Management System* »*.* Dans le cas d'un véhicule terrestre l'affichage tête-basse peut par exemple désigner un écran GPS intégré.

Un écran portable (ou transportable) 140 peut comprendre des écrans avioniques et/ou des moyens non-avioniques de type « *Electronic Flight Bag* » (ou "sac électronique") et/ou des moyens de réalité augmentée et/ou virtuelle. Selon les modes de réalisation, les affichages peuvent désigner ou comprendre des affichages en réalité virtuelle et/ou augmentée 150. De tels systèmes d'affichage peuvent en effet être portés par le pilote. L'affichage peut donc être individuel et comprendre un casque de réalité virtuelle opaque ou un casque de réalité augmentée semi-transparent (ou un casque à transparence configurable). Le casque peut donc être un *"wearable computer*", des *"glasses",* un visiocasque, etc. Les moyens de réalité virtuelle ou augmentée peuvent désigner ou comprendre des systèmes avioniques de type EVS ("*Enhanced Vision System*") ou SVS ("*Synthetic Vision System*")*.*

L'affichage dans la cabine de pilotage (l'interface homme-machine selon l'invention) peut également comprendre un ou plusieurs projecteurs 160. Un projecteur peut être par exemple un pico-projecteur ou un vidéoprojecteur (par exemple laser). Les informations affichées à destination du pilote peuvent en effet être entièrement virtuelles (affichées dans le casque individuel), ou bien entièrement réelles (par exemple projetées sur les surfaces planes disponibles dans l'environnement réel du cockpit), ou bien encore une combinaison des deux (i.e. en partie un affichage virtuel superposé ou fusionné avec la réalité et en partie un affichage réel via des projecteurs). L'utilisation de projecteurs permet en particulier de reconfigurer l'espace d'immersion du pilote (les surfaces courbes du tableau de bord peuvent être prises en compte de manière à créer à la demande un affichage fusionné global, en déformant les images projetées). La distribution de la projection d'informations et/ou de masquage peut être configurable, notamment en fonction du contexte visuel immersif de l'utilisateur. Cette "distribution" peut amener à considérer l'environnement de manière opportuniste en considérant toutes les surfaces disponibles de façon à ajouter (superposer, surimposer) des informations virtuelles, choisies de manière appropriée dans leur nature (quoi afficher), temporalité (quand afficher, à quelle fréquence) et emplacement (priorité des affichages, stabilité des emplacements versus la nature, de manière à ne pas désorienter l'utilisateur, une certaine constance peut être souhaitable). A un extrême, l'ensemble des emplacements peu ou faiblement utilisés dans l'environnement de l'utilisateur peuvent être exploités de façon à densifier l'affichage d'information. L'affichage fusionné virtuel/réel peut donc devenir très fragmenté (fonction de la complexité de l'utilisateur qui est soi dans une pièce blanche, soit dans une salle pourvue de nombreux appareillages, donc au moins une partie peut être masquée ou cachée par l'affichage d'informations acceptées ou choisies). Le réel se retrouve donc transformé en autant d'écrans "potentiels".

L'ensemble des emplacements peu ou faiblement utilisés dans l'environnement de l'utilisateur peuvent être exploités de façon à densifier l'affichage d'information. Plus encore, par projection de masques d'images superposée aux objets réels, l'affichage peut « gommer » un ou plusieurs instruments de commande présents physiquement dans le cockpit (manettes, boutons, actuateurs) dont la géométrie est connue et stable pour augmenter plus encore les surfaces adressables. L'environnement réel du cockpit peut donc se retrouver transformé en autant d'écrans "potentiels", voire en un unique écran unifié (reconfiguration du réel).

De manière optionnelle, l'affichage dans la cabine de pilotage ou de conduite de véhicule peut comprendre un ou plusieurs caméras d'acquisition d'images 170. Une caméra peut être fish-eye, stéréoscopique, ou autre. Ce retour d'images permet de nombreux développements avantageux de l'invention. Un appareil photo ou une caméra vidéo disposée dans le cockpit peuvent permettre de capturer au moins une partie de l'ensemble des informations visuelles affichées à destination du pilote (avantageusement, ce retour vidéo pourra être placé sur une visière tête haute, des *smartglasses* ou tout autre équipement porté par le pilote, de manière à capturer la vue subjective du pilote). Par analyse d'image (effectuée de manière régulière fixe ou de manière continue dans le cas d'une capture vidéo), la vue subjective du pilote peut être analysée et modifiée ou corrigée, en fonction de critères prédéfinis et/ou selon des objectifs prédéfinis.

Par exemple, dans un mode de réalisation, la densité visuelle des informations qui sont affichées peut être estimée. Par exemple, cette densité peut être estimée selon différentes sous-parties d'images et des ajustements d'affichage peuvent être déterminés de manière dynamique. Par exemple, dans le cas où un écran d'affichage deviendrait trop « encombré » (quantité de texte ou de symboles graphiques en excès par rapport à un ou plusieurs seuils prédéfinis), les informations les moins prioritaires peuvent être « réduites » ou « condensées » ou « synthétisées » sous forme de repères ou de symboles. Inversement, si la densité d'information affichée le permet, des informations réduites ou condensées ou synthétisées peuvent être développées ou détaillées ou étendues ou agrandies. Cette gestion de la présentation d'informations peut être fonction de différents paramètres (abordés ci-après).

L'affichage dans la cabine de pilotage ou de conduite de véhicule comprend un ou plusieurs dispositifs de suivi du regard 180 (« oculométrie »).

Le « suivi du regard » utilisé par le procédé selon l'invention détermine au cours du temps la position des yeux (origine du vecteur) et la direction du regard (le vecteur). Par simplification, la détermination de la position des yeux est implicite.

Des techniques d'oculométrie (en anglais « *eye-tracking* » ou « *gaze-tracking* ») peuvent en effet permettre de suivre les mouvements oculaires. Dans un mode de réalisation, un oculomètre analyse des images de l'oeil humain enregistrées par une caméra, souvent en lumière infrarouge, pour calculer la direction du regard du pilote. Dans un mode de réalisation, les variations de potentiels électriques à la surface de la peau du visage sont analysées. Dans un mode de réalisation, les perturbations induites par une lentille sur un champ magnétique sont analysées. Par oculométrie, il peut être déterminé à quel endroit se porte le regard du pilote et il peut ainsi être déterminé ce qu'il voit (et ce qu'il ne voit pas).

Différentes techniques d'oculométrie peuvent être utilisées, voire combinées.

Dans un mode de réalisation, le suivi du regard est effectué selon une technique électro-oculographique (Marg,1951), en mesurant des différences de potentiel bioélectrique, résultant du champ bioélectrique rétino-cornéen modulé par les rotations de l'oeil dans son orbite (électro-oculogrammes effectués avec des électrodes de surface).

Dans un mode de réalisation, le suivi du regard est effectué selon une technique dite du limbe (Torok et al. 1951). En éclairant le limbe de l'oeil (séparation entre la sclère et l'iris), la quantité de lumière réfléchie dépend de la surface relative de la sclère et de l'iris dans le champ de mesure, et donc permet d'identifier la position de l'oeil.

Dans un mode de réalisation, le suivi du regard est effectué selon des techniques fondées sur les principes énoncés par Hirschberg en 1985. Il est en effet possible de déterminer l'orientation du regard en repérant la position du reflet d'une source de lumière sur la cornée de l'oeil (reflet cornéen) par rapport à la pupille : une caméra peut détecter le mouvement de l'oeil explorant une image. Une analyse quantitative du mouvement oculaire peut ensuite être effectuée (nombre et durée des fixations du regard, nombre et amplitude des saccades, etc.). Cette méthode permet notamment des mesures absolues des différentes positions de l'oeil, et ce indépendamment des mouvements de la tête de l'utilisateur.

Dans un mode de réalisation, le suivi du regard est effectué par mesure des réflexions de la lumière sur les différentes structures de l'oeil (images de Purkinje ; faces intérieure et extérieure de la cornée et faces antérieure et postérieure du cristallin). Une à plusieurs caméras focalisées sur un ou les deux yeux permettent d'enregistrer/analyser leurs mouvements. Les variantes peuvent être montées sur la tête (« Head-mounted systems »). D'autres variantes sont des systèmes non-intrusifs. Ces variantes peuvent utiliser de la lumière ambiante ou de la lumière infrarouge (e.g. avec une ou plusieurs diodes). La fréquence d'acquisition varie entre 30 et des milliers de Hz. Certains systèmes non-intrusifs sont calibrés.

Dans un mode de réalisation, le suivi du regard est effectué par suivi de modèle 3D du visage. Selon cette variante, des images sont acquises afin de détecter le visage, les pupilles, la bouche et les narines. Par la suite, un modèle 3D est utilisé pour évaluer l'orientation du visage et finalement l'orientation du regard est estimée en utilisant les images des yeux.

Dans un mode de réalisation, le suivi du regard est effectué par l'approche dite « glint » (l'éclair). Utilisant la technique PCCR pour « Pupil Center/Corneal Reflection », l'angle de l'axe visuel et la localisation du regard sont déterminés en suivant la position relative de la pupille et du point de la lumière réfléchie depuis la cornée

Dans un mode de réalisation, le suivi du regard est effectué en utilisant un dispositif de type Tobii (suivi de l'oeil à distance avec une source de lumière proche de l'infrarouge, puis traitement d'images selon un modèle 3D physiologique de l'oeil pour estimer la position des yeux et la direction du regard).

L'interface homme-machine selon l'invention peut également comprendre des interfaces ou périphériques d'entrée 190. Dans un développement, le dispositif comprend des moyens de sélection d'une ou plusieurs portions de l'affichage virtuel. Les pointages des interfaces homme-machine (IHM) ou de portions de ces interfaces ou informations peuvent être accessibles via différents dispositifs, par exemple un dispositif de pointage de type « souris » ou une désignation basée sur un pointage manuel ; via des interfaces d'acquisition (bouton, molette, joystick, clavier, télécommande, capteurs de mouvement, microphone, etc.), via des interfaces combinées (écran tactile, commande à retour d'effort, gants ou *glove*, etc.). Les interfaces homme-machine d'entrée ou de sélection peuvent en effet comprendre une ou plusieurs interfaces de sélection (menus, pointeurs, etc.), interfaces graphiques, interfaces vocales, interface gestuelle et de position. Dans un mode de réalisation avantageux, une sélection peut être effectuée par le regard (e.g. durée de fixation en excès d'un seuil de durée prédéfini, clignement de l'oeil, commande vocal concomitante, contraction d'un muscle, commande au pied, etc.). Dans un mode de réalisation, une sélection peut être effectuée par un ou plusieurs mouvements de tête.

Dans un mode de réalisation, le système connaît à tout instant la direction du regard pilote et la position de ses yeux, ce qui lui permet de choisir l'affichage adéquat pour afficher les messages.

L'affichage sélectionné peut être divers (nature) et une pluralité d'espaces ou de surfaces (e.g. planes, courbes, etc.) peut être mobilisée. Un affichage peut être un écran en tête basse, un HUD, une visière du casque ou un pare-brise. Un affichage peut aussi résulter d'une projection. Dans certains modes de réalisation, les espaces de projection sont sélectionnés de manière « opportuniste » (par exemple, les espaces non utilisés du tableau de bord sont utilisés e.g. les montants ou les espaces interstitiels entre les écrans). Dans un mode de réalisation, un ou plusieurs espaces peuvent être prédéfinis pour des projections (ils peuvent être intentionnellement dédiés à cette tâche). Par exemple, une zone libre du cockpit peut permettre à un projecteur d'afficher de l'information. Généralement, rien ne restreint cette liberté de projection qui peut être effectuée sur n'importe quel type de support (matériel e.g. plastiques, tissus, verre, etc. y compris corps humain), étant donné que les systèmes de projection peuvent adapter leurs affichage de manière à se conformer à l'environnement et à produire des images stables et formées, connaissant le point de vue subjectif cible.

La figure 2 illustre un système d'affichage 200 tête-haute ou HUD.

Le type de système d'affichage optionnel qui est représenté affiche des informations (par exemple de pilotage) en superposition avec le paysage extérieur (visible par transparence ou tel que capturé et retransmis en vidéo). Ce système d'affichage 200 peut comprendre un afficheur transparent 210 sur lequel une image peut être projetée ou créée permettant de voir cette image en superposition avec une scène « extérieure ». Un système monoculaire comporte un seul afficheur. Un système binoculaire comporte deux afficheurs. Dans des variantes de réalisation la transparence d'un afficheur est variable (ou configurable). Un afficheur est fixé ou associé à la tête du pilote de manière à ce que l'afficheur maintenu proche de l'oeil (« *Near-To-Eye display* » en anglais). Dans l'exemple qui est illustré, l'afficheur est fixé à un casque porté par le pilote. D'autres moyens sont possibles (e.g. lunettes portées, support fixe duquel l'opérateur se rapproche, etc.). Dans certains mode de réalisation, un affichage HUD peut être un équipement fixé à l'avion, offrant un champ de vue fixe (en général présentant un champ de vue 40° latéral - 26° vertical). Dans d'autres modes de réalisation, l'affichage tête haute est fixé à un casque ou un appareil de vue porté par le pilote. Dans un mode de réalisation, un ou plusieurs projecteurs affichent de l'information sur le pare-brise du véhicule (e.g. avion), ou bien encore sur des zones libres du cockpit (par exemple).

Ce système d'affichage 200 peut être associé avec un système 230 permettant de détecter la direction vers/dans laquelle la tête i.e. de l'opérateur est dirigée (système de suivi du regard est utilisé (« *eye-tracking* »). Les systèmes permettant ce type de mesure sont divers (mesure optique, électrique, mécanique, etc.). Le système 230 peut être couplé à l'afficheur 200 mais il peut également être disposé ailleurs dans le cockpit en l'absence d'un casque porté par le pilote (il peut faire face au pilote, pour mesurer par exemple la dilatation et la direction des pupilles).

Le système d'affichage 200 illustré sur la figure peut comprendre ou être associé à un calculateur i.e. des ressources informatiques 240 (e.g. de calcul, de mémoire, graphique, etc.). Le calculateur 240 peut piloter (commander, asservir) le projecteur 220. Le calculateur peut exploiter l'information relative au suivi 230 de la direction de la tête et/ou du regard. Il peut intégrer et manipuler diverses informations relatives à l'aéronef et/ou la mission. Il détermine à chaque instant (en continu, selon le rafraichissement vidéo souhaité) l'image opérationnelle devant être affichée sur l'afficheur.

Le système d'affichage 200 peut être associé à une ou plusieurs interfaces homme-machine (IHM), e.g. des périphériques de saisie (souris, clavier, écran tactile, *force touch*, moyens haptiques, *trackpad*, *trackball*, etc.), permettant au pilote d'effectuer des sélections parmi plusieurs données proposées ou bien d'entrer des données. Selon les modes de réalisation, l'IHM peut comprendre différents périphériques ou combinaisons de périphériques. Dans certains cas, des commandes vocales peuvent être utilisées. Dans d'autres cas, des interfaces neuronales peuvent être utilisées. Des interfaces de sélection par clignement d'oeil peuvent être utilisées.

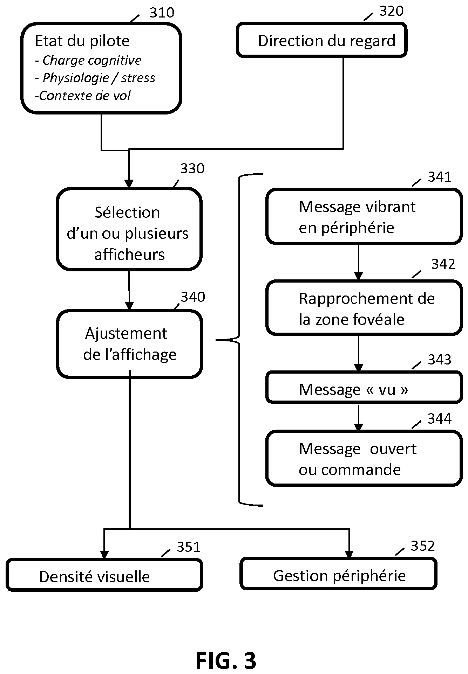

La figure 3 illustre des exemples d'étapes d'un mode de réalisation.

Dans un mode de réalisation, il est décrit un procédé mis en oeuvre par ordinateur de gestion d'une interface homme-machine comprenant une pluralité d'afficheurs, le procédé comprenant les étapes consistant à : - recevoir des informations relatives à la position des yeux et la direction du regard d'un utilisateur sur l'interface homme-machine; - recevoir des informations physiologiques de l'utilisateur ; - déterminer un niveau de charge cognitive parmi une pluralité de niveaux prédéfinis en fonction des informations physiologiques reçues ; - ajuster l'affichage de l'interface homme-machine en fonction de la direction du regard et du niveau de charge cognitive déterminée.

Une surveillance de *l'état global* du pilote peut conduire à adapter ou reconfigurer l'affichage d'une certaine manière, par exemple en densifiant ou en allégeant les écrans, etc.). L'état global du pilote peut inclure différents facteurs comme a) la charge cognitive, b) le niveau de stress corrélé à la phase de vol ou à d'autres événements ou paramètres physiques extérieurs comme le niveau sonore, c) les paramètres physiologiques ou biologiques du pilote e.g. rythme cardiaque et transpiration inférant des estimations de niveau de stress. La pondération ou la hiérarchie entre ces différents facteurs peut être statique ou évolutive/dynamique, configurable ou préconfigurée.

Dans le mode de réalisation revendiqué, le procédé comprend en outre une étape consistant à afficher en périphérie de l'interface homme-machine un message graphique à une distance configurable de l'emplacement de l'interface homme-machine sur lequel se porte le regard de l'utilisateur.

Dans un mode de réalisation, le message graphique peut être affiché sur une pluralité d'afficheurs pour suivre le regard du pilote.

Dans un mode de réalisation, le message graphique peut être consigné à un seul et même écran. Dans d'autres modes de réalisation, le message graphique peut « passer » d'afficheur en afficheur, de manière à assurer une continuité visuelle. A l'instar des autres exemples fournis, il se peut que le message suive le regard au travers des différents afficheurs, se « fige » lorsque le pilote veut le traiter. Il peut alors s'ouvrir une interface interactive au regard. Le message, une fois observé ou perçu, peut aller se placer à son emplacement par défaut (entraînement du pilote) pour être traité plus tard par ce dernier (éventuellement)..

Dans le mode de réalisation revendiqué, la distance est fonction de la charge cognitive et/ou de la priorité associée au message. Le terme « priorité » peut être remplacé par « criticité ». Plus la charge cognitive est forte et/ou plus le message est critique plus il se rapprochera du centre de la zone fovéale. Cette distance peut également se réduire au cours du temps plus le message devient critique au regard du temps.

Dans le mode de réalisation revendiqué, la distance est décroissante au cours du temps.

Dans certains cas (messages critiques), il peut être intentionnel de solliciter l'attention du pilote jusqu'à confirmation par ce dernier qu'il a accédé consciemment à l'information.

Dans un mode de réalisation avantageux de l'invention, le symbole graphique suit en effet le regard du pilote (avec changement d'afficheur si besoin) tant que celui-ci ne l'a pas perçue (et donc manifestement confirmé).

Dans un mode de réalisation, le message graphique est affiché selon des modalités graphiques comprenant des déplacements en translation et/ou des rotations, telles que des vibrations, ces déplacements et/ou rotations étant fonction du contenu du message.

Le « contenu » du message peut désigner sa priorité et/ou sa criticité.

Les vibrations (i.e. des mouvements de faible amplitude) en vision périphérique sont particulièrement avantageuses pour les raisons évoquées précédemment (propriétés de la vision périphérique).

Dans un mode de réalisation, l'étape consistant à ajuster l'affichage comprend l'étape consistant à sélectionner un ou plusieurs afficheurs parmi les afficheurs constituant l'interface homme-machine.

Dans un mode de réalisation, le procédé détermine l'afficheur privilégié à utiliser en fonction de la direction du regard afin de capter au mieux l'attention du pilote. L'afficheur le plus proche du centre fovéal peut être sélectionné.

Dans un mode de réalisation, les informations physiologiques comprennent un ou plusieurs des paramètres comprenant le rythme cardiaque, la variabilité du rythme cardiaque, le rythme respiratoire, les mouvements des yeux, les fixations du regard, la dilatation pupillaire, le taux de cortisol, la température de la peau, la conductivité de la peau, un ou plusieurs marqueurs de l'activité du système parasympathique, un signal de type électrocardiographie, un signal de type électroencéphalographie, un signal de type magnétoencéphalographie, un signal de type fNIR ou un signal de type fMRI.

Le niveau de charge cognitive peut être déterminé en fonction d'un ou de plusieurs paramètres physiologiques de l'utilisateur ou du pilote (et/ou des dynamiques de ces paramètres), mesurés physiquement et/ou estimés logiquement, directement ou indirectement. La détermination de l'état physiologique du pilote peut comprendre des mesures directes et/ou indirectes. Les mesures directes peuvent notamment comprendre une ou plusieurs mesures directes du rythme cardiaque et/ou ECG (électrocardiogramme) et/ou EEG (électroencéphalogramme) et/ou de la transpiration et/ou du rythme de la respiration du pilote. Les mesures indirectes peuvent notamment comprendre des estimations de l'excitation ou de la fatigue ou du stress du pilote, lesquels états peuvent en particulier être corrélées aux phases de vol ou à d'autres paramètres.

Les paramètres physiologiques manipulés peuvent comprendre (ordre indifférent) : le suivi du regard comprenant le suivi du mouvements des yeux et/ou les fixations du regard (en anglais « Nearest Neighbor index » ou NRI), le taux de cortisol récupéré au niveau de la salive par exemple (« Hypothalamus Pituitary Adreanal » ou HPA en anglais), le rythme cardiaque, la variabilité de ce rythme cardiaque (« Heart Rate Variability » en anglais, acronyme HRV), un ou plusieurs marqueurs de l'activité du système parasympathique, le rythme respiratoire, la température de la peau, le niveau de sudation, la conductivité de la peau (en anglais « galvanic skin response » ou GSR), la dilatation pupillaire (en anglais « pupilometry » ou « Index of Cognitive Activity (ICA) »), un signal ECG (électrocardiographie), un signal EEG (électroencéphalographie), un signal MEG (magnétoencéphalographie), un signal fNIR (en anglais « functional near-infrared imaging ») ou un signal fMRI (en anglais « functional magnetic résonance »).

Dans un mode de réalisation, le niveau de charge mentale ou cognitive « intrinsèque » ou « physiologique » est celui qui résulte de l'agrégation de données physiologiques mesurées physiquement et directement sur le pilote. Ces valeurs physiologiques peuvent être pondérées entre elles, de manière à définir un score entre des bornes prédéfinies (par exemple entre 0 et 100), éventuellement personnalisable par pilote.

Ce niveau de charge cognitive peut résulter de mesures de nature physiologiques : il peut « internaliser » l'ensemble des contraintes cognitives internes ainsi que les événements extérieurs qui sont traités par la cognition du pilote. Le niveau de charge cognitive tel que défini est donc nécessaire et suffisant pour participer de la régulation de l'affichage selon l'invention.

Dans certains modes de réalisation facultatifs, le niveau de charge cognitive est contextualisé, du fait des facteurs externes ou « extrinsèques », comme le type de tâche effectué (décollage, croisière, déroutement, révision,...) ou le contexte de vol (bruit, lumière, vibrations dans simulateur, cabine télé pilotage drone, A380, petit avion etc.),. Ces facteurs considérés isolément pour eux mêmes peuvent avantageusement mettre en perspective la charge cognitive du pilote à un moment donné, cad peuvent permettre d'en déterminer la dynamique (e.g. évolution probable, tendances historicisées, etc.). Avantageusement, la connaissance du contexte de travail permet d'anticiper, au moins de manière probabiliste, l'évolution de la charge cognitive du pilote, et par suite les adaptations de l'affichage à sa destination.

Dans un mode de réalisation, le niveau de charge cognitive est en outre déterminé en fonction de paramètres d'environnement comprenant le contexte de vol. Différents tests (donc des mesures actives, additionnelles à l'observation passive) peuvent être menés pour évaluer les données cognitives et/ou physiologiques/biologiques du pilote. Par exemple, la mesure de la charge cognitive du pilote peut être évaluée en analysant son comportement courant sur la tâche de pilotage en cours (critères prédéfinis) et/ou par des tests additionnels (opportunistes, incidents, etc.) proposés de manière facultative durant les intervalles de temps interstitiels pendant lesquels il est déterminé que le pilote peut accepter de tels tests. Ces évaluations peuvent conduire en retour à une adaptation de l'affichage.

Dans un mode de réalisation, le suivi du regard détermine, en fonction de durées de fixation prédéfinies et/ou de mouvements oculaires prédéfinis, des actions comprenant l'agrandissement du message, la réduction du message, l'envoi d'une donnée ou d'une commande de pilotage.

Dans un mode de réalisation, le procédé comprend en outre une étape consistant à acquérir au moins une image de l'interface homme-machine.

Dans un mode de réalisation, la géométrie du cockpit est connue (nombre, types et inclinaisons des écrans, etc.), par configuration manuelle ou par l'intermédiaire d'un fichier de configuration.

Dans un mode particulier de réalisation, la géométrie du cockpit peut être connue de manière semi-automatique, voire entièrement automatique. Par exemple, une boucle « retour » (par exemple en la forme d'une caméra capturant le point de vue visuel subjectif du pilote) permet de détecter le nombre, le type et la configuration de chacun des écrans présents (e.g. détourage, etc.). L'étape d'acquisition d'une image (globale) de l'interface-homme machine est avantageuse en ce qu'elle permet une configuration automatique de l'affichage.

Dans un mode de réalisation, le procédé comprend en outre une étape consistant à mesurer la densité visuelle de l'interface homme-machine et l'étape consistant à ajuster l'affichage de l'interface homme-machine étant fonction de la densité visuelle mesurée.

Dans un mode de réalisation, le procédé comprend une étape consistant à désactiver l'affichage de l'interface homme-machine après que l'utilisateur porte son regard à un emplacement prédéfini pendant une durée en excès d'une durée prédéfinie (ajustements graphiques « débrayables »).

Dans un mode de réalisation, il est décrit un système comprenant des moyens pour mettre en oeuvre une ou plusieurs des étapes du procédé, l'interface homme-machine comprenant - une pluralité d'afficheurs choisis parmi un affichage tête-haute 120, un affichage tête-basse 130, un écran portable ou transportable 140, un affichage en réalité virtuelle et/ou augmentée 150, un ou plusieurs projecteurs 160, une caméra ou dispositif d'acquisition d'images 170 ; - un dispositif de suivi du regard de l'utilisateur de l'interface homme-machine.

Dans un mode de réalisation, le système comprend en outre un casque de réalité augmentée et/ou de réalité virtuelle.

Dans un mode de réalisation, le système comprend en outre une boucle retour de régulation en la présence d'une caméra pour l'acquisition d'une image au moins approximative de la vue subjective par l'utilisateur de l'interface homme-machine.

Dans un mode de réalisation, l'affichage se caractérise par l'application de règles d'affichage prédéfinies, lesquelles sont fonction de i) l'état global du pilote 310 *(incluant une pondération entre a) charge cognitive*, *b) niveau de stress*, *c) phase de vol activité en cours*, *paramètres d'environnement extérieurs internalisés au sens cognitif)* et/ou de ii) la direction du regard 320.

L'ajustement de l'affichage 330 peut se faire de différentes manières. Dans un mode de réalisation, l'affichage est modifié (implication directe). Dans un mode de réalisation, les règles gouvernant la gestion de l'affichage sont affectées (implication indirecte via un changement dans les règles de régulation).

Dans un développement, l'affichage dans l'interface homme-machine est régi par des règles prédéfinies, ces règles comprenant des règles d'emplacement d'affichage et des règles de priorité d'affichage. La cartographie de ces interfaces homme-machine est définie en fonction de la configuration réelle d'implémentation (simulateur, type d'avion, type de mission). Par exemple, un utilisateur (ou un instructeur ou une compagnie aérienne) peut prédéfinir manuellement les différentes zones d'espace auxquelles afficher tel ou tel type d'informations. Tout ou partie des écrans et des interfaces homme-machine associées peut être transposée ou déportée à l'intérieur d'un espace virtuel ou de réalité augmentée (le cas échéant). Des règles de substitution d'images ou de flux d'images sont possibles. Certaines règles peuvent être associées ou prévoir des priorités d'affichage différentes, des durées minimales et maximales d'affichage, des affichages permanent, intermittent, régulier ou irrégulier, des affichages optionnels et remplaçables, des affichages non-désactivables, des modalités ou paramètres d'affichage (luminance, surface, texture, etc.)

Dans un développement, les règles d'emplacement sont prédéfinies. Dans un mode de réalisation les interfaces homme-machine peuvent être configurées dans et pour un cockpit spécifique par l'instructeur selon ses préférences personnelles. Dans un mode de réalisation, cette configuration peut être entièrement manuelle. Par exemple, l'instructeur peut prévoir l'affichage dans le casque de fenêtres dédiées à l'instruction et ne gênant pas la vision des élèves. Ces fenêtres peuvent être virtuellement attachées au cockpit: par exemple, l'instructeur peut configurer le contenu de ces fenêtres (paramètres de l'aéronef, paramètres relatifs aux pilotes, suivi des performances, etc.). La définition de l'espace virtuel peut donc être associée aux caractéristiques géométriques du cockpit ou de du simulateur de vol.

De manière générale, l'affichage d'un ou de plusieurs symboles peut être optimisé (i.e. adapté, par exemple à la révision en cours et/ou au contexte de vol). Spécifiquement, le modèle d'interaction sélectionné (traduit par l'affichage de symboles graphiques correspondants) peut être réparti sur les différents écrans de manière optimisée (e.g. distribution ou répartition spatiale des informations sur les différents écrans disponibles et/ou accessibles). Par exemple, en matière d'espace, l'affichage peut être réparti ou fractionné ou distribué entre plusieurs dispositifs d'affichage, le cas échéant. Par exemple, optionnellement, le procédé peut décaler ou déplacer graphiquement, par exemple durant le temps de la saisie, l'ensemble de l'affichage pour permettre au modèle de substitution de s'afficher aux limites de cette zone d'affichage.

L'affichage de la valeur peut par exemple s'effectuer à différents endroits dans le champ visuel du pilote, par exemple à proximité du moyen de révision (doigt, curseur) ou à d'autres endroits dans le cockpit (projection tête haute, surimpression de type réalité augmentée, restitution 3D etc.). En matière temporelle, le symbole graphique peut comprendre des séquences d'affichage ordonnées de diverses manières.

Dans un mode de réalisation, un retour d'informations (par exemple de saisie clavier) peut être affiché dans le champ périphérique. Dans un mode de réalisation, la visualisation du retour d'information est conditionnée au fait que le regard de l'utilisateur se fixe et/ou se dirige vers une certaine zone prédéfinie.

Selon ce mode de réalisation, *a contrario*, le retour d'informations reste en zone périphérique si le parcours du regard ne satisfait pas à des conditions prédéfinies (e.g. selon des critères de temps et/ou d'espace). Dans un mode de réalisation, le retour d'informations peut « suivre » le regard, de manière à rester dans un état dans lequel il peut être sollicité, mais sans pour autant obstruer l'emplacement courant du regard.

A cette fin, le champ visuel peut être « discrétisé » selon différentes zones. Qualitativement, les zones peuvent être qualifiées (par exemple) en zone d'obstruction frontale, zone d'attraction de l'attention forte, modérée, faible, nulle, etc. Quantitativement, ces zones peuvent être déterminées de manière chiffrée ou objective (périmètres d'espace, distances précises avec tolérances, intervalles de confiance, etc.). Les zones discrétisées du champ visuel peuvent être déterminées de manière « universelle » (pour un groupe d'utilisateurs), ou de manière personnalisée et individuelle.

Dans un mode de réalisation, la densité visuelle 341 est ajustée et/ou l'affichage en champ de vision périphérique 342 est ajusté.

La « densité visuelle » peut être manipulée. La densité visuelle peut être mesurée en nombre de pixels allumés ou actifs par centimètre carré, et/ou en nombre de caractères alphanumériques par unité de surface et/ou en nombre de motifs géométriques prédéfinis par unité de surface. La densité visuelle peut également être définie, au moins partiellement, selon des critères physiologiques (modèle de vitesse de lecture par le pilote, etc.).

Dans un mode de réalisation de l'invention, cette densité visuelle peut être maintenue sensiblement constante. Dans d'autres modes de réalisation, la densité visuelle sera ajustée. Le contexte de vol par exemple peut moduler cette densité visuelle (par exemple, à l'atterrissage ou dans les phases critiques du vol, la densité d'informations peut être volontairement réduite ou à l'inverse un maximum d'informations peut être affiché).

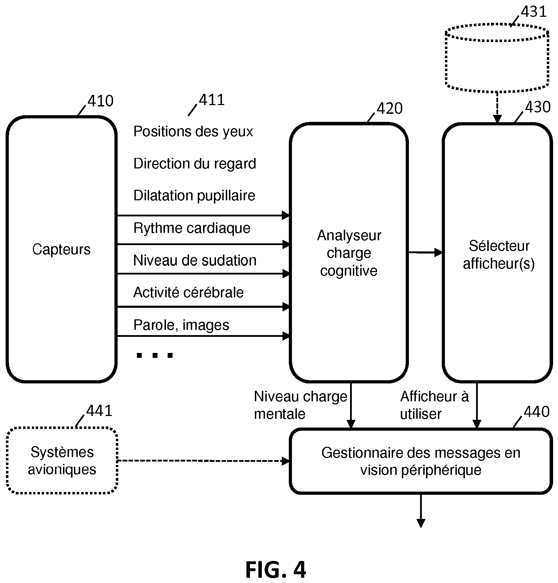

La figure 4 détaille un mode de réalisation particulier, concernant la gestion du champ de vision périphérique.

La charge cognitive du pilote 420 est déterminée à partir de capteurs 410.

Un ou plusieurs paramètres peuvent conditionner la sélection 430 d'un ou plusieurs afficheurs (120, 130, 140, 150), éventuellement en connaissant la géométrie de la cabine de pilotage 431 ou les équipements RV/RA disponibles.

Dans un mode de réalisation, c'est la direction du regard qui conditionne la sélection de l'affichage, et non la charge cognitive. La charge cognitive quant à elle conditionne alors la distance d'affichage des messages par rapport au centre du cône de vision.

Dans un mode de réalisation, la charge cognitive seule conditionne la sélection de l'affichage. Par exemple, dans certaines situations de saturation ou d'urgence, l'essentiel des écrans peut être éteint et un seul écran peut être mobilisé (quelle que soit la direction du regard).

Dans un mode de réalisation, les deux facteurs interviennent (à parts égales ou selon différentes pondérations ou compromis). La direction du regard *et* la charge cognitive influe directement ou indirectement sur la sélection de l'affichage (un ou plusieurs afficheurs).

Par suite, un gestionnaire de messages en vision périphérique 440 en interaction avec les systèmes avioniques 441 affiche un ou plusieurs messages sur les affichages ou systèmes d'affichages sélectionnés (120, 130, 140, 150, 160).

Les capteurs 410 sont des capteurs, notamment physiologiques, directs ou indirects, mesurant des paramètres physiques ou chimiques. Les capteurs 410 peuvent notamment comprendre des appareils d'oculométrie (e.g. positions des yeux, mouvements, direction du regard, dilatation pupillaire, détection des clignements et de leur fréquence, etc.), des dispositifs de mesure de paramètres physiques de l'environnement (e.g. luminosité ambiante, hygrométrie, niveau sonore, CO2 exhalé, etc.), et des dispositifs de mesure physiologique (e.g. rythme cardiaque ; rythme respiratoire ; EEG ; ECG ; niveau de sudation e.g. nuque, main ; humidité oculaire ; mouvement du corps du pilote, tête, mains, pieds, etc.).

L'ensemble des signaux bruts est centralisé et analysé dans une logique, dénommée « analyseur de charge cognitive » 420, lequel interprète et catégorise l'ensemble des paramètres mesurés en catégories prédéfinies, notamment selon des niveaux de « charge cognitive » discrétisées (e.g. « charge cognitive maximale », « charge cognitive en croissance rapide », « charge cognitive nulle (sommeil) », etc.).

La discrétisation des états de charge cognitive peut être modulée ou atténuée ou nuancée par de nombreux autres paramètres facultatifs additionnels, par exemple selon des niveaux de stress eux aussi quantifiés (e.g. « stress absent » ou « stress maximal (atterrissage) ». Une charge cognitive intense peut ainsi se dérouler en absence de stress ou au contraire dans un état de stress maximal. Par ailleurs, le contexte de vol peut favoriser ou inhiber le stress et/ou la charge cognitive (qui différent selon les phases de vol décollage, montée, croisière, déroutement, révision du plan de vol, atterrissage, roulage, etc.).

Les différentes catégories ainsi obtenues peuvent chacune être associées avec des règles de gestion de l'affichage de messages 440.

La gestion de l'affichage peut être effectuée en fonction de plusieurs objectifs, notamment des critères d'attention. Le cerveau humain, bien que percevant correctement les signaux visuels (à la limite sans apparition d'une rivalité oculaire), peut en effet « occulter » des éléments d'information, dans certaines circonstances. Un pilote concentré sur un objet présent à l'horizon peut abstraire des objets présents au premier plan, éventuellement dangereux. Cette rivalité attentionnelle, étant exclusive et propre à chaque cerveau, ne peut pas être mesurée ni inférée (« boîte noire »). En revanche, des rappels (et leurs variations, pour éviter les accoutumances) peuvent être judicieusement mis en oeuvre, pour permettre *in fine* de soutenir l'attention de l'utilisateur (placé dans une situation de risque ou de décision par hypothèse, justifiant un maintien dans un état d'éveil ou de conscience particulier).

Selon les modes de réalisation de l'invention, tout ou partie des afficheurs disponibles sera sollicitée.

Dans un mode de réalisation particulier, un seul écran est sélectionné, en fonction de la direction du regard. Avantageusement, la sélection d'un seul écran (par exemple celui qui se trouve à plus courte distance de l'emplacement courant du regard) permet une interaction rapide et naturelle (au sens qu'elle minimise les perturbations ou les changements visuels). Un des avantages remarquables de l'invention réside dans le fait de s' « abstraire » des afficheurs et de suivre le regard pour que le pilote puisse interagir rapidement.

Dans un mode de réalisation particulier, un ou plusieurs d'écrans (« afficheurs », « affichages ») sont sélectionnés, en fonction de la charge cognitive ou mentale déterminée et/ou de la direction du regard.

La sélection d'afficheurs 430 peut être effectuée de différentes manières.

Dans un mode de réalisation, la sélection d'afficheurs peut notamment tirer parti de la connaissance de la géométrie de la cabine de pilotage (surfaces des différents écrans, leurs formes, leurs positions dans l'espace, lesquels sont le plus fréquemment consultés par le pilote, où est la zone fovéale, etc.). Cette configuration évoluant peu, cette solution à configuration initiale peut être suffisante et satisfaisante.

Dans un mode de réalisation, cette connaissance de la géométrie peut être déterminée ou atteinte par l'emploi d'une capture d'image en vue subjective (par exemple une caméra vidéo montée sur le casque porté par le pilote peut permettre d'approcher ce qu'il perçoit de son environnement, agencé de multiples écrans). Par suite, la région d'affichage souhaitée peut être mise en correspondance avec un écran particulier (adressage). Ce développement confère de la flexibilité (et ne requiert pas nécessairement de calibrage manuel).

Dans un mode particulier de réalisation, l'affichage de l'interface homme-machine peut tenir compte de la position de l'opérateur et/ou de la direction du regard, afin notamment d'être toujours présenté conformément à la géométrie de la cabine ou selon une vue subjective optimisée.

Dans un mode particulier de réalisation, l'existence d'une boucle retour vidéo peut être particulièrement avantageuse, en ce qu'elle peut permettre, notamment couplée avec l'emploi de projecteurs de redéfinir l'environnement visuel de l'utilisateur de façon dynamique, par exemple en s'adaptant à l'environnement (selon des méthodes automatisées ou automatiques).

L'utilisation en combinaison du dispositif de suivi du regard de l'interface homme-machine selon l'invention est particulièrement avantageuse, en ce qu'elle permet de moduler ou réguler ou influencer les règles d'emplacement et/ou règles de priorité d'affichage.

Dans certains modes de réalisation avantageux (e.g. en cas de tunnel cognitif), les écrans trop distants sont éteints. Des variantes prévoient la diminution progressive de la densité visuelle (pour minimiser les perturbations à l'égard du pilote).

Concernant la gestion des messages en vision périphérique 440, de nombreux modes de réalisation sont possibles.

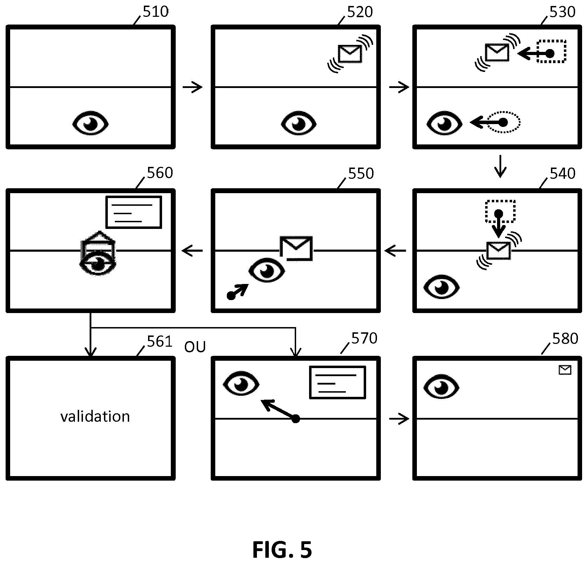

La figure 5 illustre un exemple de gestion de l'affichage dans le champ de vision périphérique.

Connaissant l'état global du pilote (cognition interne), les paramètres extérieurs (phase de vol, priorité d'un message), le procédé selon l'invention comprend une étape consistant à recevoir un message depuis les systèmes avioniques (e.g. alerte météorologique, consigne de vol, instruction ATC, déroutement, etc.). Ce message peut être associé à un niveau de priorité ou de criticité variable.

Le procédé peut arbitrer l'affichage, en fonction de facteurs comprenant l"état cognitif du pilote (charge mentale) et la direction de son regard.

Un mode de réalisation tout à fait spécifique et qui ne serait être limitatif est décrit ci-après. A l'étape 510, le regard du pilote est déterminé comme portant à un emplacement donné dans l'interface homme-machine. Dans un mode de réalisation, un message de priorité en excès d'un seuil prédéfini est émis par les services avioniques : ce message requiert l'attention du pilote à brève échéance. Dans un autre mode de réalisation, l'état de conscience du pilote nécessite de maintenir ou remonter son attention: un message spécifique doit stimuler le pilote.

Par suite, à l'étape 520, un message est affiché en périphérie. Dans un mode de réalisation avantageux, ce message *vibre.* Dans un mode de réalisation, le message vibre selon des modalités qui sont fonctions de la criticité prédéfinie associée au message (la forme dépend du fond). Les vibrations (i.e. des mouvements de faible amplitude) en vision périphérique sont particulièrement avantageuses pour les raisons évoquées précédemment (propriétés de la vision périphérique).

A l'étape 530, le pilote poursuit son activité, qu'il ait ou non perçu (visuellement) le message affiché en périphérie : son regard se déplace. Le message, au lieu de rester en place, se déplace corrélativement au déplacement du regard (selon différentes modalités : de manière proportionnelle, homothétique, etc.). Cette étape 530 attire progressivement l'attention du pilote.

A l'étape 540, le pilote regarde toujours ailleurs i.e. ne consulte toujours pas le message. La gestion de l'affichage rapproche alors intentionnellement l'affichage du symbole/icône de l'emplacement actif du regard du pilote. Dans un mode de réalisation, la distance spatiale entre le message vibrant et l'emplacement du regard peut notamment être *fonction* de la charge cognitive du pilote. Dans un mode de réalisation, le message se rapproche de la vision fovéale lors qu'un niveau charge mentale prédéfini a été atteint. Dans un mode de réalisation, la distance peut être fonction de cette charge cognitive *et de la criticité du message.* La fonction gouvernant la distance peut être une fonction mathématique (analytique) mais peut également être de nature algorithmique (résultat d'une série d'étapes, non formulable de manière analytique). La fonction peut par exemple diminuer la distance au cours du temps. Selon les cas, la diminution peut être effectuée à vitesse constante, ou s'accélérer.

A l'étape 550, le pilote prend conscience du message, il le regarde et le « voit ». A cet instant, les vibrations du message cessent. Dit autrement, si le regard se dirige vers le message, ce dernier se fige (les vibrations disparaissent) pour permettre l'ouverture du message et son traitement.

Différents modes de réalisation sont possibles.

Plusieurs modalités existent pour déterminer qu'un objet graphique (tel un message) a été « vu », et plus généralement pour contrôler de l'interface homme-machine (sélection d'un objet affiché). Ces modalités peuvent être combinées.

Dans un mode de réalisation, il est déterminé si le parcours du regard du pilote (i.e. de sa projection sur le plan ou l'espace de l'interface homme-machine) traverse celui d'une zone prédéfinie (par exemple autour d'un objet particulier de l'interface). La zone peut être définie strictement, i.e. selon des contours stricts mais également selon des marges de tolérance (e.g. zones tampons progressives, etc.). Ces marges peuvent être associées aux erreurs de mesure du suivi du regard. Il peut être déterminé un ou plusieurs points d'intersection entre une ou plusieurs zones de l'interface homme-machine et le parcours du regard (ou simplement son emplacement) à un instant donné. Ces points d'intersection peuvent à leur tour déclencher une ou plusieurs actions (e.g. confirmation d'envoi d'une réponse, autre affichage, etc.). Dans un mode de réalisation, la durée durant laquelle le regard du pilote traverse une zone prédéfinie associée à un message est un paramètre manipulable par le procédé selon l'invention.

Dans un mode de réalisation, le chemin oculaire ou parcours du regard croise (« intersecte », « passe sur ») le message; cette condition exclusivement spatiale, nécessaire et suffisante, détermine que le message a été vu. Ce mode de réalisation présente l'avantage de ne pas perturber l'activité « naturelle » du pilote. Dans un mode de réalisation, les conditions sont spatiales et temporelles : une durée *minimale* prédéfinie est requise (le pilote doit rester quelques fractions de seconde sur le message afin que ce dernier soit considéré comme vu). Dans un mode de réalisation, la durée de croisement est *maximale* (si le regard s'attarde sur une zone donnée, une action différente peut être requise). Dans un mode de réalisation, la durée du croisement doit être comprise dans un intervalle prédéfini (soit absolu i.e. quel que soit le message, soit relatif au type de message ou à son contenu), entre une durée minimale et une durée maximale.

A l'étape 560, le regard du pilote se porte en excès d'une durée prédéterminée sur le message, le message est alors « ouvert ». Par exemple, une surface d'affichage supérieure est utilisée, adjacente au symbole initial.

Plusieurs interactions sont ensuite possibles. A l'étape 561, le pilote peut regarder la réponse à envoyer et la valider avec un moyen physique pour sécuriser l'interaction.

Par exemple, dans le domaine aéronautique, pour des opérations critiques telles que des interactions avec le contrôle de la navigation aérienne (ATC), comme un envoi de message de type datalink, une confirmation physique ou haptique peut être requise (par convention). Pour la conduite de véhicule terrestre, partiellement automatisée, de manière analogue, certains actes physiques peuvent être requis (e.g. pour des dépassements, etc.)

Le pilote peut également contrôler - par le regard - une action préconfigurée dans ou par le message (e.g. confirmation d'une commande, sélection dans une liste, fermeture d'une fenêtre, confirmation, infirmation, etc.). Dans une autre suite, le pilote peut simplement ignorer le message à l'étape 570, lequel sera alors « réduit » à l'étape 580 (par exemple à un endroit habituel ou invariant dans l'espace défini par l'interface homme-machine). Par réduction, on peut entendre que le message est miniaturisé (la surface d'affichage est réduite) ou qu'un symbole le représentant lui est substitué. D'autres modalités sont possibles. Des combinaisons d'interactions sont possibles : par exemple, après une confirmation 561, le message peut également être réduit en champ périphérique.

En d'autres termes, recevant une demande d'alerter ou d'informer le pilote, le procédé selon l'invention peut afficher une icône dédiée dans le champ périphérique du pilote à une distance fonction de la charge cognitive courante du pilote (plus elle est élevée, plus proche sera l'icône ; cette position pouvant varier entre 10° et 30° par exemple). Dans un mode de réalisation, le procédé selon l'invention peut comprendre une étape consistant à définir l'afficheur le plus proche de la ligne horizontale associé à l'emplacement courant du regard (e.g. un écran en tête basse, un HUD, la visière d'un casque, le pare-brise, etc.). Cette caractéristique est avantageuse (« seamless display »), i.e. minimisant les changements graphiques ou les effets de surprise. La sélection de l'afficheur peut par exemple être effectuée selon une base de données détenant des informations sur la position des afficheurs dans le cockpit (et leur forme, par exemple). Le symbole ou l'icône affiché peut vibrer, afin de capter l'attention du pilote. Une vibration ou oscillation est avantageuse car l'homme est plus sensible aux mouvements qu'aux formes ou aux couleurs en vision périphérique. Dans un mode de réalisation, l'icône peut « suivre » le regard du pilote (e.g. déplacements mimétiques ou proportionnels ou homothétiques). Le suivi peut se dérouler jusqu'à ce qu'une durée prédéfinie se soit écoulée et/ou jusqu'à ce que le pilote l'ait regardée (par exemple selon le niveau de criticité de l'information). Une fois « regardé », un message interactif plus concret ou étendu ou riche ou détaillé peut apparaître. Le pilote peut traiter le message ultérieurement ; le cas échéant, le message peut se placer à la position qui lui est initialement réservée. Dans le cas où le pilote regarde effectivement le message, il peut en lire les informations et choisir d'actionner certaines fonctions contenues dans le message, par exemple en regardant des boutons dédiés à ces actions (donc par contrôle du regard). L'interface homme-machine selon l'invention peut être pilotée par le regard et/ou des interfaces d'entrée physiques (e.g. *buzzer*, souris, tactile, commande vocale): l'interface peut être exclusivement contrôlable par le regard, ou exclusivement contrôlable par des moyens physiques ou bien encore par une combinaison des deux types de contrôle (i.e. regard et action physique)

Il est décrit un produit programme d'ordinateur, ledit programme d'ordinateur comprenant des instructions de code permettant d'effectuer une ou plusieurs des étapes du procédé, lorsque ledit programme est exécuté sur un ordinateur.

A titre d'exemple d'architecture matérielle adaptée à mettre en oeuvre l'invention, un dispositif peut comporter un bus de communication auquel sont reliés une unité centrale de traitement ou microprocesseur (CPU, acronyme de « Central Processing Unit » en anglais), lequel processeur peut être "multi-core" ou "many-core"; une mémoire morte (ROM, acronyme de « Read Only Memory » en anglais) pouvant comporter les programmes nécessaires à la mise en oeuvre de l'invention; une mémoire vive ou mémoire cache (RAM, acronyme de « Random Access Memory » en anglais) comportant des registres adaptés à enregistrer des variables et paramètres créés et modifiés au cours de l'exécution des programmes précités ; et une interface de communication ou E/S (I/O acronyme de « Input/ouput » en anglais) adaptée à transmettre et à recevoir des données. Dans le cas où l'invention est implantée sur une machine de calcul reprogrammable (par exemple un circuit FPGA), le programme correspondant (c'est-à-dire la séquence d'instructions) peut être stocké dans ou sur un médium de stockage amovible (par exemple une carte SD, ou un stockage de masse tel que un disque dur e.g. un SSD) ou non-amovible, volatile ou non-volatile, ce médium de stockage étant lisible partiellement ou totalement par un ordinateur ou un processeur. La référence à un programme d'ordinateur qui, lorsqu'il est exécuté, effectue l'une quelconque des fonctions décrites précédemment, ne se limite pas à un programme d'application s'exécutant sur un ordinateur hôte unique. Au contraire, les termes programme d'ordinateur et logiciel sont utilisés ici dans un sens général pour faire référence à tout type de code informatique (par exemple, un logiciel d'application, un micro logiciel, un microcode, ou toute autre forme d'instruction d'ordinateur, comme des web services ou SOA ou via des interfaces de programmation API) qui peut être utilisé pour programmer un ou plusieurs processeurs pour mettre en oeuvre des aspects des techniques décrites ici. Les moyens ou ressources informatiques peuvent notamment être distribués ("Cloud computing"), éventuellement avec ou selon des technologies de pair-à-pair et/ou de virtualisation. Le code logiciel peut être exécuté sur n'importe quel processeur approprié (par exemple, un microprocesseur) ou coeur de processeur ou un ensemble de processeurs, qu'ils soient prévus dans un dispositif de calcul unique ou répartis entre plusieurs dispositifs de calcul (par exemple tels qu'éventuellement accessibles dans l'environnement du dispositif). Des technologies de sécurisation (crypto-processeurs, authentification éventuellement biométrique, chiffrement, carte à puce, etc) peuvent être utilisées.

## Revendications

1. Procédé mis en oeuvre par ordinateur de gestion d'une interface homme-machine comprenant une pluralité d'afficheurs, comprenant les étapes consistant à :
- recevoir des informations relatives à la position des yeux et la direction du regard d'un utilisateur sur l'interface homme-machine;
- recevoir des informations physiologiques de l'utilisateur ;
- déterminer un niveau de charge cognitive parmi une pluralité de niveaux prédéfinis en fonction des informations physiologiques reçues ;
- ajuster l'affichage de l'interface homme-machine en fonction de la direction du regard et du niveau de charge cognitive déterminée ; et
afficher en périphérie de l'interface homme-machine un message graphique à une distance configurable de l'emplacement de l'interface homme- machine sur lequel se porte le regard de l'utilisateur, le message provenant d'un système avionique et concernant un paramètre de vol délivrée par le système avionique, la distance étant fonction de la charge cognitive et/ou de la priorité associée au message et un emplacement du message graphique étant déplacé de manière à ce que la distance soit décroissante au cours du temps.

2. Procédé selon l'une quelconque des revendications précédentes, le message graphique étant affiché selon des modalités graphiques comprenant des déplacements en translation et/ou des rotations, telles que des vibrations, ces déplacements et/ou rotations étant fonction du contenu du message.

3. Procédé selon la revendication 1, l'étape consistant à ajuster l'affichage comprenant l'étape consistant à sélectionner un ou plusieurs afficheurs parmi les afficheurs constituant l'interface homme-machine.

4. Procédé selon la revendication 1, les informations physiologiques comprenant un ou plusieurs paramètres comprenant le rythme cardiaque, la variabilité du rythme cardiaque, le rythme respiratoire, les mouvements des yeux, les fixations du regard, la dilatation pupillaire, le taux de cortisol, la température de la peau, la conductivité de la peau, un ou plusieurs marqueurs de l'activité du système parasympathique, un signal de type électrocardiographie, un signal de type électroencéphalographie, un signal de type magnétoencéphalographie, un signal de type fNIR ou un signal de type fMRI.

5. Procédé selon la revendication 1, le niveau de charge cognitive étant en outre déterminé en fonction de paramètres d'environnement comprenant le contexte de vol.

6. Procédé selon l'une quelconque des revendications précédentes, le suivi du regard déterminant, en fonction de durées de fixation prédéfinies et/ou de mouvements oculaires prédéfinis, des actions comprenant l'agrandissement du message, la réduction du message, l'envoi d'une donnée ou d'une commande de pilotage.

7. Procédé selon l'une quelconque des revendications précédentes, comprenant en outre une étape consistant à acquérir au moins une image de l'interface homme-machine.

8. Procédé selon l'une quelconque des revendications précédentes, comprenant en outre une étape consistant à mesurer la densité visuelle de l'interface homme-machine et l'étape consistant à ajuster l'affichage de l'interface homme-machine étant fonction de la densité visuelle mesurée.

9. Produit programme d'ordinateur, ledit programme d'ordinateur comprenant des instructions de code permettant d'effectuer les étapes du procédé selon l'une quelconque des revendications 1 à 8, lorsque ledit programme est exécuté sur un ordinateur relié à un dispositif de suivi du regard d'un utilisateur d'un interface homme-machine.

10. Système comprenant des moyens pour mettre en oeuvre les étapes du procédé selon l'une quelconque des revendications 1 à 8, l'interface homme-machine comprenant :
- une pluralité d'afficheurs choisis parmi un affichage tête-haute 120, un affichage tête-basse 130, un écran portable ou transportable 140, un affichage en réalité virtuelle et/ou augmentée 150, un ou plusieurs projecteurs 160, une caméra ou dispositif d'acquisition d'images 170 ;
- un dispositif de suivi du regard de l'utilisateur de l'interface homme-machine
- un processeur configuré pour exploiter des informations relatives à une position des yeux et une direction d'un regard de l'utilisateur de l'interface homme-machine transmise par le dispositif de suivi du regard de l'utilisateur de l'interface homme-machine et configuré pour piloter ladite pluralité d'afficheurs.

11. Système selon la revendication 10, comprenant en outre un casque de réalité augmentée et/ou de réalité virtuelle.

12. Système selon l'une quelconque des revendications 10 à 11, comprenant en outre une boucle retour de régulation en la présence d'une caméra pour l'acquisition d'une image au moins approximative de la vue subjective par l'utilisateur de l'interface homme-machine.

## Patentansprüche

1. Computerimplementiertes Verfahren zum Verwalten einer Mensch-Maschine-Schnittstelle, die eine Vielzahl von Anzeigegeräten umfasst, umfassend die Schritte bestehend aus:
- Empfangen von Informationen betreffend die Position der Augen und die Blickrichtung eines Benutzers auf der Mensch-Maschine-Schnittstelle,
- Empfangen von physiologischen Informationen des Benutzers;
- Bestimmen eines kognitiven Belastungslevels aus einer Vielzahl von vorgegebenen Leveln als Funktion der empfangenen physiologischen Informationen;
- Einstellen des Anzeigegeräts der Mensch-Maschine-Schnittstelle als Funktion der Blickrichtung und des bestimmten kognitiven Belastungslevels; und
Anzeigen, in der Peripherie der Mensch-Maschine-Schnittstelle, einer graphischen Nachricht in einem konfigurierbaren Abstand von der Lage der Mensch-Maschine-Schnittstelle, auf die der Blick des Benutzers gerichtet ist, wobei die Nachricht von einem Luftfahrtsystem stammt und einen Flugparameter betrifft, der von dem Luftfahrtsystem bereitgestellt wird, wobei der Abstand eine Funktion der kognitiven Belastung und/oder der der Nachricht zugeordneten Priorität ist und eine Lage der graphischen Nachricht so verschoben wird, dass der Abstand im Laufe der Zeit abnimmt.

2. Verfahren nach einem der vorhergehenden Ansprüche, wobei die graphische Nachricht gemäß den graphischen Modalitäten angezeigt wird, umfassend Translationsverschiebungen und/oder Drehungen, wie Vibrationen, wobei diese Verschiebungen und/oder Drehungen eine Funktion des Inhalts der Nachricht sind.

3. Verfahren nach Anspruch 1, wobei der Schritt, der darin besteht, das Anzeigegerät einzustellen, den Schritt umfasst, der darin besteht, ein oder mehrere Anzeigegeräte von den Anzeigegeräten auszuwählen, die die Mensch-Maschine-Schnittstelle darstellen.

4. Verfahren nach Anspruch 1, wobei die physiologischen Informationen einen oder mehrere Parameter umfassen, umfassend den Herzrhythmus, die Variabilität des Herzrhythmus, den Atemrhythmus, Augenbewegungen, Fixierungen des Blicks, die Pupillenerweiterung, den Cortisolspiegel, die Hauttemperatur, die Hautleitfähigkeit, einen oder mehrere Kennungsmarken der Aktivität des parasympathischen Systems, ein Elektrokardiogrammsignal, ein Elektroenzephalogrammsignal, ein Magnetenzephalogrammsignal, ein fNIR- oder ein fMRI-Signal.

5. Verfahren nach Anspruch 1, wobei das kognitive Belastungslevel ferner als Funktion von Umweltparametern bestimmt wird, die den Flugkontext umfassen.

6. Verfahren nach einem der vorhergehenden Ansprüche, wobei das Folgen des Blicks als Funktion von vorgegebenen Dauern der Fixierung und/oder von vorgegebenen Augenbewegungen Aktionen bestimmt, die die Vergrößerung der Nachricht, die Verkleinerung der Nachricht, das Senden eines gegebenen Datenstücks oder eines Steuerbefehls umfassen.

7. Verfahren nach einem der vorhergehenden Ansprüche, ferner umfassend einen Schritt, der darin besteht, mindestens ein Bild der Mensch-Maschine-Schnittstelle zu erfassen.

8. Verfahren nach einem der vorhergehenden Ansprüche, ferner umfassend einen Schritt, der darin besteht, die visuelle Dichte der Mensch-Maschine-Schnittstelle zu messen, und der Schritt, der darin besteht, das Anzeigegerät der Mensch-Maschine-Schnittstelle einzustellen, eine Funktion der gemessenen visuellen Dichte ist.

9. Computerprogrammprodukt, wobei das Computerprogramm Code-Befehlezum Ausführen der Schritte des Verfahrens nach einem der Ansprüche 1 bis 8 umfasst, wenn das Programm auf einem Computer ausgeführt wird, der mit einer Vorrichtung zum Folgen des Blicks eines Benutzers einer Mensch-Maschine-Schnittstelle verbunden ist.

10. System, umfassend Mittel zum Ausführen der Schritte des Verfahrens nach einem der Ansprüche 1 bis 8, wobei die Mensch-Maschine-Schnittstelle Folgendes umfasst:
- eine Vielzahl von Anzeigegeräten, ausgewählt aus einem Head-up-Anzeigegerät 120, einem Head-down-Anzeigegerät 130, einem tragbaren oder transportierbaren Bildschirm 140, einem Anzeigegerät mit virtueller und/oder erweiterter Realität 150, einem oder mehreren Projektoren 160, einer Kamera oder einer Bilderfassungsvorrichtung 170;
- eine Vorrichtung zum Folgen des Blicks des Benutzers der Mensch-Maschine-Schnittstelle;
- einen Prozessor, der dafür konfiguriert ist, Informationen betreffend eine Position der Augen und eine Blickrichtung des Benutzers der Mensch-Maschine-Schnittstelle, die von der Vorrichtung zum Folgen des Blicks des Benutzers der Mensch-Maschine-Schnittstelle übertragen wird, auszuwerten, und der dafür konfiguriert ist, die Vielzahl von Anzeigegeräten zu steuern.

11. System nach Anspruch 10, ferner umfassend einen Helm mit erweiterter und/oder virtueller Realität.

12. System nach einem der Ansprüche 10 bis 11, ferner umfassend eine Rückkopplungsregelschleife in Gegenwart einer Kamera zum Erfassen eines Bildes zumindest annähernd des subjektiven Blicks durch den Benutzer der Mensch-Maschine-Schnittstelle.

## Claims

1. A computer-implemented method for managing a human-machine interface comprising a plurality of displays, comprising the steps of:
- receiving information relating to the position of the eyes and the direction of the gaze of a user on the human-machine interface;
- receiving physiological information of the user;
- determining a level of cognitive load from among a plurality of predefined levels on the basis of the received physiological information;
- adjusting the display of the human-machine interface on the basis of the direction of the gaze and of the determined level of cognitive load; and
displaying, at the periphery of the human-machine interface, a graphical message at a configurable distance from the location of the human-machine interface at which the gaze of the user is directed, the message originating from an avionic system and relating to a flight parameter delivered by the avionic system, the distance being dependent on the cognitive load and/or the priority associated with the message and a location of the graphical message being moved so that the distance decreases over time.

2. The method according to any one of the preceding claims, the graphical message being displayed according to graphical techniques comprising translational movements and/or rotations, such as vibrations, these movements and/or rotations being dependent on the content of the message.

3. The method according to claim 1, the step of adjusting the display comprising the step of selecting one or more displays from among the displays forming the human-machine interface.

4. The method according to claim 1, the physiological information comprising one or more parameters comprising heart rate, heart rate variability, breathing rate, eye movements, gaze fixations, pupil dilation, cortisol level, skin temperature, skin conductivity, one or more markers of the activity of the parasympathetic system, an electrocardiography signal, an electroencephalography signal, a magnetoencephalography signal, an fNIR signal or an fMRI signal.

5. The method according to claim 1, the level of cognitive load further being determined on the basis of environment parameters comprising the flight context.

6. The method according to any one of the preceding claims, the gaze tracking determining, on the basis of predefined fixation durations and/or of predefined ocular movements, actions comprising enlarging the message, reducing the message, sending an item of data or a piloting command.

7. The method according to any one of the preceding claims, further comprising a step of acquiring at least one image of the human-machine interface.

8. The method according to any one of the preceding claims, further comprising a step of measuring the visual density of the human-machine interface and the step of adjusting the display of the human-machine interface being dependent on the measured visual density.

9. A computer program product, said computer program comprising code instructions for performing the steps of the method according to any one of claims 1 to 8 when said program is executed on a computer linked to a device for tracking the gaze of a user of a human-machine interface.

10. A system comprising means for implementing the steps of the method according to any one of claims 1 to 8, the human-machine interface comprising:
- a plurality of displays chosen from among a head-up display 120, a head-down display 130, a portable or transportable screen 140, a virtual and/or augmented reality display 150, one or more projectors 160, a camera or an image acquisition device 170;
- a device for tracking the gaze of the user of the human-machine interface
- a processor, configured to use information relating to a position of the eyes and a direction of a gaze of the user of the human-machine interface transmitted by the device for tracking the gaze of the user of the human-machine interface and configured to steer said plurality of displays.

11. The system according to claim 10, further comprising an augmented reality and/or virtual reality headset.

12. The system according to any one of claims 10 to 11, further comprising an adjustment feedback loop in the presence of a camera for acquiring an at least approximate image of the subjective view for the user of the human-machine interface.
